# FASCICULE DE BREVET EUROPEEN

(11) **EP 2 863 977 B1**
(45) Date de publication et mention de la délivrance du brevet: **04.05.2016**
(21) Numéro de dépôt: 13727890.9
(22) Date de dépôt: 07.06.2013
(51) Int. Cl.: A61M 16/04, A61M 16/06

(54) **CANULE OROPHARYNGÉE COMPRENANT UNE ARRIVÉE DE DIOXYGÈNE ET UNE EXTRACTION DE DIOXYDE DE CARBONE**
MUND- UND RACHENKANÜLE MIT EINEM SAUERSTOFFEINLASS UND EINEM KOHLENDIOXIDAUSLASS
OROPHARYNGEAL CANNULA COMPRISING AN OXYGEN INLET AND A CARBON DIOXIDE OUTLET

(30) Priorité: 21.06.2012 FR 1255869
(43) Date de publication de la demande: 29.04.2015
(73) Titulaire: Deltamedics, 60270 Gouvieux (FR)
(72) Inventeur: OZENNE, Jean-Christophe, 60270 Gouvieux (FR)
(74) Mandataire: Casalonga
(86) Numéro de dépôt international: PCT/EP2013/061826
(87) Numéro de publication internationale: WO 2013/189763

(56) Documents cités:
- US-A- 1 498 810
- US-A- 5 273 032
- US-A- 6 098 617
- US-A1- 2008 000 481
- US-A1- 2008 110 456
- US-A1- 2009 275 851
- US-A1- 2010 030 027
- US-A1- 2010 198 096

## Description

L'invention concerne un dispositif de libération des voies aériennes d'un patient, plus particulièrement une canule oropharyngée permettant une injection de dioxygène ainsi qu'une mesure capnographique.

Une canule est un tube droit ou recourbé permettant le passage d'un fluide tel que de l'air ou un liquide à travers un orifice. Une canule oropharyngée, telle qu'une canule de Guedel ou canule de Mayo, est utilisée en médecine pour maintenir ouvertes les voies aériennes d'un patient inconscient, par exemple un patient sous anesthésie ou un patient plongé dans un coma.

Une canule de Guedel se présente sous la forme d'un tube semi-rigide en matière plastique comportant trois parties.

Une première partie est incurvée de manière à suivre le trajet de la cavité buccale jusqu'au pharynx. Sa forme permet notamment d'avancer le massif lingual, éloignant celui-ci de la paroi postérieure du pharynx, et de libérer ainsi un canal entre la langue et le palais du patient jusqu'à son pharynx. De cette manière, un passage d'air est maintenu vers les poumons, et la langue est maintenue en position pour ne pas qu'elle s'affaisse vers l'arrière sur l'épiglotte et n'obstrue les voies respiratoires, notamment lorsque le patient inconscient est allongé sur le dos.

Une seconde partie droite est armée à l'intérieur d'un anneau rigide destinée à être au niveau de la zone dentaire. La rigidité de cette zone permet de conserver l'orifice ouvert lorsque la canule est placée entre les dents en évitant qu'un patient intubé n'obstrue par exemple un tube orotrachéal par morsure.

Une troisième partie comprend une collerette qui repose sur les lèvres du patient une fois la canule en place.

Il existe plusieurs tailles de canule de Guedel. Les tailles varient en fonction de la distance entre la commissure labiale et l'angle de la mâchoire chez l'enfant et l'adulte. Chez l'adulte, on peut aussi utiliser la distance comprise entre la commissure des lèvres et le lobe de l'oreille.

Lors d'une opération sous anesthésie, un masque facial est généralement utilisé pour injecter du dioxygène au patient via le conduit de la canule de Guedel, et les narines du patient. L'utilisation d'un masque gêne cependant l'accès au visage du patient en cas de besoin.

De plus, l'utilisation d'un masque entraîne une mise en place complexe pour pouvoir réaliser une capnographie de l'air expiré par le patient.

La capnographie est une mesure de la concentration ou de la pression partielle de dioxyde de carbone dans l'air expiré par un patient. De telles mesures sont très utilisées sur des patients sous anesthésie. La présence de dioxyde de carbone dans l'air expiré sur plusieurs expirations par un patient venant d'être intubé permet notamment de confirmer que le tube endotrachéal est bien dans la trachée.

La capnographie permet par ailleurs d'obtenir une mesure indirecte de la pression partielle de dioxyde de carbone dans le sang artériel. Cette information permet d'évaluer l'état de vascularisation du patient. La capnographie reflète de manière directe la capacité d'élimination du dioxyde de carbone par les poumons du patient, et de manière indirecte, la production de dioxyde de carbone par les tissus et son transport jusqu'aux poumons.

Elle permet de détecter très tôt des signes de déficiences respiratoires telles qu'une hypoventilation, ou une déconnexion d'un circuit ou d'un tube dans l'oesophage. Lors d'une opération sous anesthésie, la capnographie permet de fournir des informations, telles que la fréquence et la régularité de ventilation, plus utiles que celle fournies par un oxymètre.

Elle fournit une méthode de détection rapide de conditions critiques, comme un tube trachéal mal positionné, un défaut de ventilation, un défaut circulatoire, et de prévenir des complications irréversibles.

Pour réaliser à la fois une injection de dioxygène et un prélèvement des gaz expirés par le patient pour réaliser une mesure du taux de dioxyde de carbone en s'acquittant de l'utilisation d'un masque facial gênant, il est connu du document US 2007/0095347 un dispositif nasopharyngé adapté. Cependant, un tel dispositif ne peut être utilisé que par voie nasale.

Il est également connu des documents US 2007/267024, US 2008/308108 et EP 1 188 457 un dispositif adapté pour former un passage de gaz à inhaler dans lequel est logée une paire de conduits adaptés pour être glissés dans le ou les passage(s) interne(s) pour injecter du dioxygène, sur une portion proximale notamment, et prélever du gaz expiré, sur une portion distale notamment, pour mesurer le taux de CO₂.

Ces dispositifs présentent cependant l'inconvénient d'être en plusieurs parties, une partie composée de la canule et une partie composée des tubes d'injection et d'extraction à insérer. Le temps supplémentaire d'assemblage peut être critique en situation d'urgence notamment.

Il est également connu du document US 2008/0000481 un dispositif oropharyngé de forme adaptée pour être inséré dans la bouche d'un patient. Le dispositif comprend un corps comportant une portion distale, une portion proximale et au moins deux canaux s'étendant de la portion proximale à la portion distale pour former deux passages d'air au travers desquels peut être injecté du dioxygène d'une part, et peut être extrait du dioxyde de carbone d'autre part. Les canaux sont formés dans le corps du dispositif qui comprend une collerette sur la portion proximale pour empêcher que le dispositif ne soit introduit trop profondément dans la bouche.

Ces dispositifs oropharyngés ne présentent pas de moyens de fixation simple et rapide sur le patient permettant son maintien sur le patient une fois le dispositif installé dans la bouche du patient, tout en permettant une extraction rapide du dispositif dans le cas où le patient se réveille ou dans un cas où une intubation est nécessaire.

En effet, une canule oropharyngée doit être installée et fixée sur le patient de sorte que celui-ci puisse la retirer au besoin, notamment dans le cas d'un réveil du patient. La fixation de la canule permet de maintenir en position le dispositif, mais cette fixation doit être amovible facilement. En effet, les canules de Guedel peuvent provoquer un vomissement chez un patient conscient. Un patient reprenant conscience va spontanément recracher la canule lorsqu'il retrouvera le réflexe de la toux. Elle peut aussi être remplacée à un moment par un dispositif d'intubation.

L'invention vise à pallier les inconvénients mentionnés ci-dessus en fournissant une canule oropharyngée adaptée pour être fixée sur le patient notamment à l'aide des tubes d'injection/extraction la canule en place sur le patient.

Selon un aspect de l'invention, il est proposé une canule oropharyngée comprenant un corps formé d'une partie distale incurvée pour être insérée dans la bouche d'un patient et bloquer sa langue et d'une partie proximale comprenant une portion droite couplée à une collerette apte à reposer sur les lèvres du patient, un conduit principal formant un passage de fluide entre la partie proximale et la partie distale du corps, et deux conduits auxiliaires formés dans le corps et s'étendant de la partie proximale jusque dans le conduit principal.

Selon une caractéristique générale de l'invention, chaque conduit auxiliaire débouche dans la collerette sur un orifice d'entrée/sortie orienté selon une direction radiale par rapport à l'axe du conduit principal de manière à être orienté latéralement de part d'autre de la bouche du patient lorsque la canule est installée sur le patient.

En orientant ainsi les orifices d'entrée/sortie, les tubes d'injection/extraction couplés aux orifices d'entrée/sortie s'étendent en direction des oreilles du patient, de chaque côté de la bouche du patient. De cette manière chaque tube d'injection/extraction peut être passé derrière une oreille pour sécuriser la canule oropharyngée sur le patient de manière facilement amovible.

De préférence, la direction entre chaque orifice d'entrée/sortie et l'axe du conduit principal forme un angle compris entre 0° et 30°, et plus particulièrement entre 10° et 20°, avec un axe passant par les commissures des lèvres lorsque la canule est installée sur le patient.

Un angle compris entre 10 et 20°, notamment un angle de 15°, permet de diriger les tubes d'injection/extraction vers le rebord supérieur des oreilles, et ainsi faciliter le passage des tubes derrière les oreilles.

La collerette peut comprendre un premier et un second coudes d'entrée/sortie solidaires chacun à une extrémité d'un conduit auxiliaire, chaque coude comprenant un desdits orifices d'entrée/sortie de la collerette à l'extrémité opposée.

Dans ce mode de réalisation, la collerette peut comporter n'importe quelle épaisseur. Les coudes d'entrée/sortie sont en saillie à partir de la surface de la collerette.

Selon une alternative, chaque conduit auxiliaire peut comprendre une portion formée radialement dans la collerette, l'orifice d'entrée/sortie étant disposé radialement sur la collerette.

Dans ce mode de réalisation, l'épaisseur de la collerette doit posséder une épaisseur suffisante pour permettre le passage des conduits auxiliaires.

Avantageusement, la canule oropharyngée peut comprendre deux tubes souples d'injection/extraction aptes à être passés derrière les oreilles du patient lorsque la canule est installée sur le patient, une première extrémité de chaque tube étant connectée à un orifice d'entré/sortie.

La connexion des tubes sur les orifices d'entrée sortie peut être réalisée de manière amovible ou bien solidaire et inamovible. La présence des tubes solidaires sur la canule oropharyngée permet d'éviter un quelconque assemblage à part la connexion des extrémités libres des tubes à des moyens d'extraction, comme un capnographe, ou des moyens d'injection lors de l'installation de la canule sur le patient.

La canule oropharyngée peut avantageusement comprendre une bague de serrage apte à maintenir les deux tubes ensemble et à coulisser le long des deux tubes de manière à maintenir la canule en place lorsqu'elle est installée sur le patient et que chaque tube passe derrière une oreille.

La bague de serrage permet, une fois les tubes passés derrière chaque oreille de serrer le tube sous le menton en remontant la bague de serrage vers le menton. Cela permet d'assurer une fixation sécurisée de la canule et tout en permettant d'enlever rapidement la canule en cas de besoin.

Avantageusement, une seconde extrémité des tubes d'injection/extraction peut comprendre des moyens de connexion à des appareils d'injection ou d'extraction.

Les moyens de connexion peuvent être des connectiques spécifiques dédiées à des appareils de mesure ou d'injection de gaz. En ayant une connectique spécifique pour chaque tube, les risques d'erreur de branchement sont nuls, même en situation d'urgence, car chaque connectique est dédiée à un appareil spécifique.

De préférence, le premier conduit auxiliaire est destiné à l'injection de dioxygène dans les voies respiratoires du patient, et le second conduit auxiliaire est destiné à l'extraction de gaz expirés par le patient pour réaliser une mesure capnographique.

Le second conduit auxiliaire débouche préférentiellement dans la portion distale, et le premier conduit auxiliaire débouche dans la portion proximale à l'opposée de la collerette.

En séparant les lieux où débouchent les conduits auxiliaires dans le conduit principal, les perturbations liées notamment aux turbulences créées par l'injection de dioxygène sont réduites pour l'extraction des gaz expirés par le patient, et la mesure de dioxyde de carbone comprend un meilleur rapport signal sur bruit. D'après les mesures empiriques et la mécanique des fluides, il est préférable de réaliser une extraction des gaz expirés dans une zone plus distale que l'injection d'oxygène, de manière à être moins gêné par le flux d'oxygène.

Dans une variante, on peut également avoir deux tubes de prélèvement de dioxyde de carbone couplés aux deux orifices d'entrée sortie, les deux tubes étant couplés à un même dispositif de capnographie. De cette manière, le dispositif peut n'être utilisé que pour l'échantillonnage du dioxyde de carbone. Dans cette configuration, les deux conduits auxiliaires peuvent déboucher dans le conduit principal à des endroits similaires ou séparés. De manière similaire, la canule oropharyngée peut être utilisée uniquement pour de l'injection de dioxygène via les deux conduits auxiliaires.

De préférence, le premier conduit auxiliaire est formé dans une paroi supérieure de la partie distale du corps, et le second conduit auxiliaire est formé dans une paroi inférieure de la partie distale du corps.

En réalisant le second conduit auxiliaire dans la paroi inférieure de la partie distale, on réduit les risques d'occlusion par sécrétions, comme les glaires et la salive. En effet, lorsque le patient est allongé sur le dos la surface inférieure de la paroi distale de la canule oropharyngée installée dans la bouche du patient se trouve surélevée au niveau de l'extrémité libre par rapport au fond de la gorge où peuvent s'accumuler les sécrétions.

Chaque conduit auxiliaire peut avantageusement comprendre une section de forme oblongue au moins sur la partie distale du corps.

De cette façon, le volume de fluide, gazeux notamment, pouvant être acheminé dans le conduit est plus important que dans le cas d'un conduit cylindrique en section. De plus, avec un corps comprenant un conduit principal de section oblongue en section, c'est-à-dire similaire à une forme de bouche, les conduits auxiliaires peuvent être réalisés dans la paroi supérieure et la paroi inférieure sans avoir besoin d'augmenter de façon importante l'épaisseur des parois du corps de la canule. Et ce tout en conservant un volume de gaz transféré important.

Avantageusement, la canule peut comprendre au moins un conduit supplémentaire formé dans le corps et s'étendant depuis la collerette jusqu'à au moins la partie distale du corps. Le conduit supplémentaire peut être destiné à l'aspiration de sécrétion dans le pharynx du patient de manière à éviter que les voies respiratoires ne soient obstruées par ces sécrétions. L'aspiration des sécrétions peut également être effectuée via un tube inséré dans le conduit principal.

La canule oropharyngée peut avantageusement comprendre également un conduit supplémentaire formé dans la collerette s'étendant du premier conduit auxiliaire jusqu'à deux orifices supplémentaires de sortie réalisés dans une partie supérieure de la collerette, les deux orifices supplémentaires de sortie étant disposés de manière à être en regard de chaque narine du patient lorsque la canule est installée sur la patient.

De préférence, au moins un des conduits auxiliaires comprend un orifice débouchant dans le conduit principal possédant une forme tronconique avec une plus grande section que la section moyenne du conduit auxiliaire.

La forme tronconique de l'orifice débouchant sur le conduit principal au niveau de la portion tubulaire du corps permet de favoriser la bonne répartition du dioxygène injecté dans un cas, et d'optimiser l'aspiration des sécrétions dans un autre cas.

Chaque portion de conduit auxiliaire s'étendant radialement dans la collerette forme un angle compris entre 0° et 20° et plus particulièrement un angle d'environ 10° avec le plan défini par la surface de la collerette de manière à orienter les orifices d'entrée/sortie vers le visage du patient.

Cet angle permet d'orienter les tubes d'injection/extraction de manière à les maintenir au plus près du visage du patient, et ainsi de réduire les risques qu'un élément ou un outil se prenne dans les tubes d'injection/extraction.

D'autres avantages et caractéristiques de l'invention apparaîtront à l'examen de la description détaillée d'un mode de réalisation, nullement limitatif, et des dessins annexés, sur lesquels :
- la figure 1 représente, de manière schématique, une vue de dessous d'une canule oropharyngée selon un mode de réalisation de l'invention ;
- la figure 2 illustre une vue en coupe selon un plan transversale II-II' de la canule oropharyngée de la figure 1 ;
- la figure 3 illustre une vue en coupe selon un plan transversale III-III' de la canule oropharyngée de la figure 1;
- la figure 4 illustre une vue en coupe selon un plan transversale IV-IV' de la canule oropharyngée de la figure 1;
- la figure 5 illustre une vue en coupe selon un plan transversale V-V' de la canule oropharyngée de la figure 1 ;
- la figure 6 illustre une vue en coupe selon un plan transversale VI-VI' de la canule oropharyngée de la figure 1;
- la figure 7 présente un schéma d'une canule oropharyngée installée sur un patient selon un autre mode de réalisation.

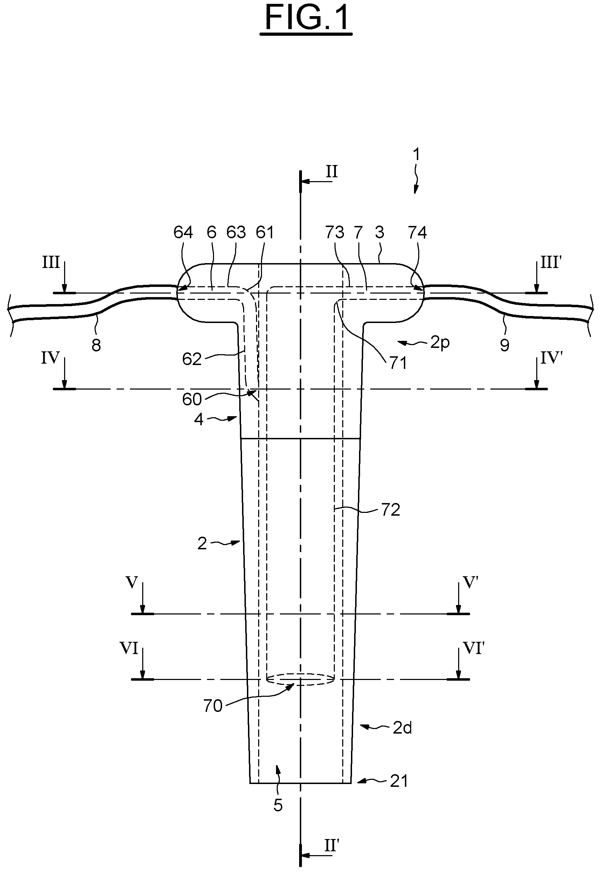

Sur la figure 1 est représentée schématiquement une vue de dessous d'une canule oropharyngée 1 selon un mode de réalisation de l'invention.

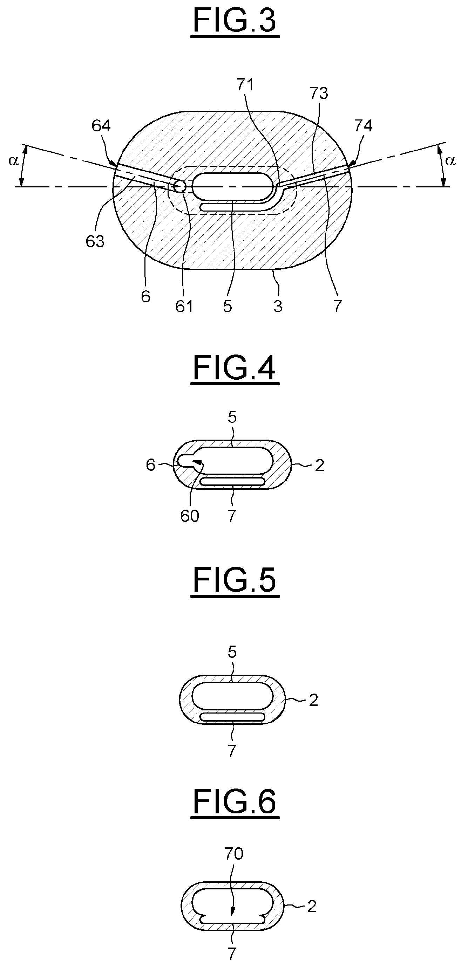

La canule oropharyngée comprend un corps 2 en matériau plastique rigide formé d'une partie proximale 2p et d'une partie distale 2d. Comme cela est illustré sur la figure 4 présentant une coupe transversale selon le plan II-II' de la canule de la figure 1, la partie distale 2d possède une forme incurvée. Cette partie est incurvée de manière à permettre l'insertion de la canule oropharyngée, et notamment sa partie distale dans la bouche d'un patient et de bloquer le massif lingual en avant et ainsi maintenir le pharynx ouvert.

La partie proximale comprend une collerette 3 et une portion droite 4 disposée entre la collerette et la partie distale 2d. La collerette 3 est destinée à reposer sur les lèvres du patient une fois la canule insérée dans la bouche du patient, tandis que la portion droite 4 se trouve au niveau des dents du patient. Le corps comprend un conduit principal 5 s'étendant de la collerette 3 de la portion proximale 2p jusqu'à l'extrémité libre 21 de la partie distale 2d en passant par la portion droite 4. Le conduit principal 5 forme ainsi un passage fluidique entre la partie proximale 2p et la partie distale 2d du corps 2 de la canule oropharyngée 1. Une fois installé sur ce patient, le conduit principal 5 forme un passage fluidique entre le pharynx et les lèvres du patient.

La portion droite 4 peut être réalisée en matériau plus rigide que le reste du corps 2 de la canule 1 de manière à renforcer la zone contre tout risque de morsure qui pourrait entraîner l'obstruction du conduit principal 5. Pour renforcer la portion droite 4, un manchon, non représenté, peut également être inséré dans le conduit principal 5 au travers de la collerette 3 de manière à s'étendre entre la collerette 3 et la portion droite 4, le manchon étant creux en son axe central pour maintenir un passage fluidique dans le conduit principal 5.

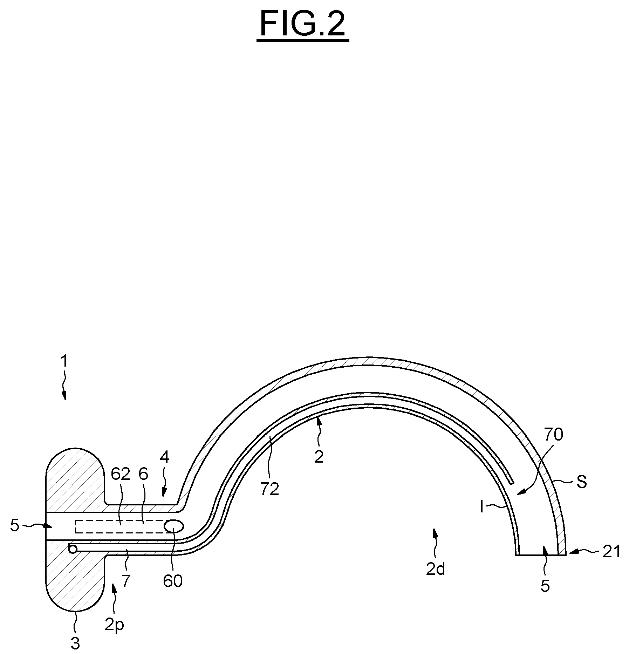

Comme cela est illustré sur les figures 1 et 2, la canule oropharyngée 1 comprend un premier conduit auxiliaire 6 formé dans l'épaisseur du corps 2 séparant le conduit principal 5 de l'extérieur du corps 2. Le premier conduit auxiliaire 6 s'étend de la collerette 3 à l'extrémité de la portion droite 4 connectée à la partie distale 2d. Il débouche dans le conduit principal 5 via une première ouverture 60, comme cela est représenté sur la figure 1 ainsi que sur la figure 4 qui représente une vue selon le plan de coupe IV-IV' de la canule oropharyngée 1 de la figure 1.

Comme représenté sur la figure 1 et sur la figure 3 qui représente une vue en coupe selon plan III-III' de la canule oropharyngée 1 de la figure 1, le premier conduit auxiliaire 6 comprend un coude 61 joignant une première portion 62 du premier conduit auxiliaire 6 s'étendant parallèlement à l'axe II-II' jusqu'à la première ouverture 60 et une seconde portion 63 du premier conduit auxiliaire 6 s'étendant dans la collerette 3. La seconde portion 63 s'étend dans la collerette 3 selon une direction radiale par rapport à l'axe II-II' jusqu'à un orifice d'entrée 64.

La canule oropharyngée 1 comprend un second conduit auxiliaire 7 également formé dans l'épaisseur du corps 2 et ayant une forme oblongue en section. Le second conduit auxiliaire 7 traverse le corps 2 depuis la collerette 3 et débouche dans le conduit principal 5 via une seconde ouverture 70 disposée en amont de l'extrémité libre 21 de la partie distale 2d, comme cela est représenté sur les figures 1 et 2 ainsi que sur la figure 6 qui représente une vue selon le plan de coupe VI-VI' de la canule oropharyngée de la figure 1. En positionnant ainsi la seconde ouverture 70 en amont de l'extrémité libre 21 de la portion distale, on réduit les risques d'obstruction de la seconde ouverture 70 par des sécrétions dans la gorge.

Ces risques sont encore plus limités de part le choix de réaliser le second conduit auxiliaire 70 dans la paroi inférieure *I* de la partie distale 2d. En effet, lorsque le patient est allongé sur le dos la paroi inférieure *I* de la paroi distale 2d de la canule oropharyngée 1 installée dans la bouche du patient se trouve surélevée au niveau l'extrémité libre 21 par rapport au fond de la gorge où peuvent s'accumuler les sécrétions.

Pour réduire d'autant plus l'éventuelle accumulation de sécrétions dans la gorge, la canule oropharyngée 1 peut comprendre un conduit supplémentaire, non représenté, formé dans le corps 2 et s'étendant de la collerette 3 à l'extrémité libre 21 de la partie distale 2d. Le conduit supplémentaire est préférentiellement réalisé dans la paroi supérieure S de la partie distale 2d de manière à être en contact avec le fond de la gorge où s'accumulent les sécrétions. Le conduit supplémentaire est couplé à des moyens d'aspiration de manière à éliminer les sécrétions.

Comme représenté sur les figures 1 à 3, le second conduit auxiliaire 7 comprend un coude 71 joignant une première portion 72 du second conduit auxiliaire 7 s'étendant selon l'axe II-II' jusqu'à la seconde ouverture 70 et une seconde portion 73 du second conduit auxiliaire 7 s'étendant dans la collerette 3. La seconde portion 73 du second conduit auxiliaire 7 s'étend dans la collerette 3 selon une direction radiale par rapport à l'axe II-II' jusqu'à un orifice de sortie 74.

Le premier conduit auxiliaire 6 est destiné à délivrer un flux de dioxygène. Le second conduit auxiliaire 7 est destiné à prélever une portion des gaz expirés par le patient pour mesurer le taux de dioxyde de carbone dans les gaz expirés grâce à un capnographe.

Pour cela, l'orifice d'entrée 64 et l'orifice de sortie 74 sont respectivement connectés à un tube d'injection 8 et un tube d'extraction 9 comme cela est illustré sur la figure 1. La connexion est réalisée par soudure ou par surmoulage de la collerette 3 sur les tubes d'injection et d'extraction 8 et 9, ou encore à l'aide de connectique.

L'extrémité libre du tube d'injection 8 comprend une connectique spécifique , par exemple un raccord conique standard pour dioxygène, destinée à être couplée à un dispositif délivrant un flux de dioxygène, optionnellement via un réducteur de section de tube. L'extrémité libre du tube d'injection 9 comprend en revanche une connectique spécifique, par exemple un raccord de type « luer lock », destinée à être couplée à un capnographe.

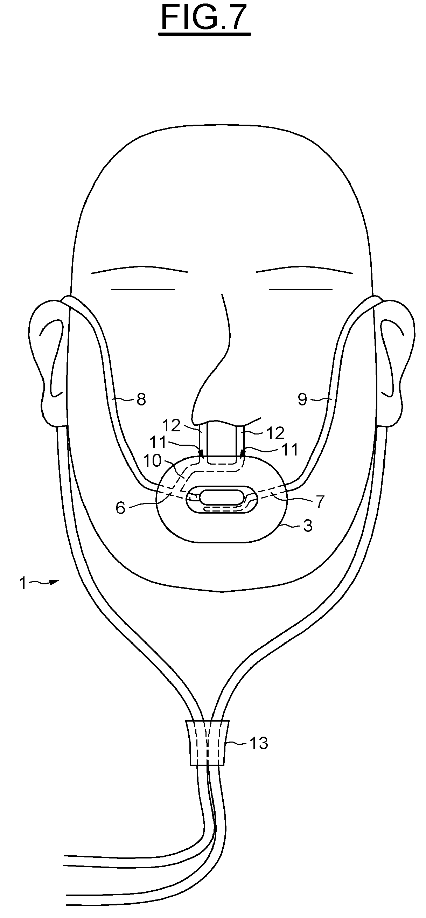

Comme cela est illustré sur la figure 3, la seconde portion 63 du premier conduit auxiliaire 6 et la seconde portion 73 du second conduit auxiliaire 7 forment chacune un angle α avec un axe passant par le premier coude 61 et le second coude 62. L'angle α possède une valeur d'environ 15°. Cet angle permet au tube d'injection 8 et au tube d'extraction 9 d'être chacun orientés en direction du rebord supérieur d'une oreille du patient de manière à faciliter le passage des tubes d'injection et d'extraction 8 et 9 derrière les oreilles, comme cela est illustré sur la figure 7.

De plus la seconde portion 63 du premier conduit auxiliaire 6 et la seconde portion 73 du second conduit auxiliaire 7 peuvent former chacune un angle d'environ 10° avec le plan défini par la surface de la collerette 3 de manière à orienter les tubes d'injection et d'extraction 8 et 9 vers le visage du patient et optimiser ainsi le maintien en position de la canule oropharyngée 1.La figure 7 présente un schéma d'une canule oropharyngée installée sur un patient selon un autre mode de réalisation. Le passage des tubes d'injection et d'extraction 8 et 9 derrière les oreilles du patient permet de maintenir la canule oropharyngée 1 en place sur les lèvres du patient. Dans ce mode de réalisation la canule oropharyngée 1 comprend en plus une bague de serrage 13 couplée aux tubes d'injection et d'extraction 8 et 9. La bague de serrage 13 est montée coulissante le long des tubes d'injection et d'extraction 8 et 9 de manière à permettre de remonter la bague 13 jusqu'à ce que les tubes d'injection et d'extraction soient serrés sous le menton du patient de sorte que la canule oropharyngée 1 soit maintenu de manière sécurisée en position sur le patient.

Dans ce mode de réalisation, la canule 1 comprend également des sorties supplémentaires d'injection de dioxygène par voie nasale. La canule 1 comprend un conduit supplémentaire 10 formé dans la collerette 3. Le conduit supplémentaire 10 est raccordé au premier conduit auxiliaire 6 et s'étend dans la collerette jusqu'à deux orifices supplémentaires de sortie 11. Les orifices supplémentaires de sortie 11 sont réalisés sur une partie supérieure radiale de la collerette 3 de manière à être en regard des narines du patient lorsque la canule1 est installée. Les deux orifices supplémentaires de sortie 11 sont couplés à deux tubes supplémentaires 12 courts insérés dans les narines du patient lorsque la canule 1 est installée pour injecter du dioxygène dans les narines du patient.

De manière à améliorer l'évacuation de l'humidité présente dans le tube d'extraction 9, le tube d'extraction peut comprendre une portion de quelques centimètre, 5 cm par exemple, de tube en Nafion. Cette portion est de préférence située à 2 cm de la collerette 3.

L'invention proposée fournit une canule oropharyngée apte à être installée et fixée rapidement sur le patient de manière amovible tout en étant maintenue de manière sécurisée en position sur le patient. De plus, la canule oropharyngée proposée est réalisée en une seule pièce ou est préassemblée de manière à ce qu'il n'y ait plus qu'à connecter les tubes d'injection/extraction aux appareils destinés. Cela permet de réduire le temps d'installation et les risques d'erreur de branchement.

## Revendications

1. Canule oropharyngée (1) comprenant un corps (2) formé d'une partie distale (2d) incurvée pour être insérée dans la bouche d'un patient et bloquer sa langue et d'une partie proximale (2p) comprenant une collerette (3) apte à reposer sur les lèvres du patient, un conduit principal (5) formant un passage de fluide entre la partie proximale (2p) et la partie distale (2d) du corps (2), et deux conduits auxiliaires (6, 7) formés dans le corps (2) et s'étendant de la partie proximale (2p) jusque dans le conduit principal (5), **caractérisée en ce que** chaque conduit auxiliaire (6, 7) débouche dans la collerette (3) sur un orifice d'entrée/sortie (64, 74) orienté selon une direction radiale par rapport à l'axe (II-II') du conduit principal (5) de manière à être orienté latéralement de part d'autre de la bouche du patient lorsque la canule (1) est installée sur le patient.

2. Canule (1) selon la revendication 1, dans laquelle la direction entre chaque orifice d'entrée/sortie (64, 74) et l'axe (II-II') du conduit principal (5) forme un angle compris entre 0° et 30°, et plus particulièrement entre 10° et 20°, avec un axe passant par les commissures des lèvres lorsque la canule (1) est installée sur le patient.

3. Canule (1) selon l'une des revendications 1 ou 2, dans laquelle chaque conduit auxiliaire (6, 7) comprend un coude d'entrée/sortie couplé à la collerette (3), chaque conduit auxiliaire (6, 7) traversant la collerette (3) jusque dans le coude d'entrée/sortie débouchant sur l'orifice d'entrée/sortie (64, 74).

4. Canule (1) selon l'une des revendications 1 ou 2, dans laquelle chaque conduit auxiliaire (6, 7) comprend une portion (63, 73) formée radialement dans la collerette (3), l'orifice d'entrée/sortie (64, 74) étant disposé radialement sur la collerette (3).

5. Canule (1) selon l'une des revendications 1 à 4, comprenant deux tubes souples d'injection/extraction (8, 9) aptes à être passés derrières les oreilles du patient lorsque la canule (1) est installée sur le patient, une première extrémité de chaque tube étant connectée à un orifice d'entré/sortie (G4, 74).

6. Canule (1) selon la revendication 5, comprenant une bague de serrage apte à maintenir les deux tubes (8, 9) ensemble et à coulisser le long des deux tubes (8, 9) de manière à maintenir la canule (1) en place lorsqu'elle est installée sur le patient et que chaque tube (8, 9) passe derrière une oreille.

7. Canule (1) selon l'une des revendications 5 ou 6, dans laquelle une seconde extrémité des tubes d'injection/extraction (8, 9) comprend des moyens de connexion à des appareils d'injection ou d'extraction.

8. Canule (1) selon l'une des revendications 1 à 7, dans laquelle le premier conduit auxiliaire (6) comprend un orifice d'entrée (64) connecté à un tube d'injection (8) de dioxygène dans les voies respiratoires du patient, et le second conduit auxiliaire (7) comprend un orifice de sortie (74) connecté à un tube d'extraction (9) de gaz expirés par le patient pour réaliser une mesure capnographique.

9. Canule (1) selon la revendication 8, dans laquelle le second conduit auxiliaire (7) débouche dans la portion distale (2d), et le premier conduit auxiliaire (6) débouche dans la portion proximale (2p) à l'opposée de la collerette (3).

10. Canule (1) selon la revendication 1 à 8, dans laquelle le premier conduit auxiliaire (6) est formé dans une paroi supérieure (S) de la partie distale (2d) du corps (2), et le second conduit auxiliaire (7) est formé dans une paroi inférieure (I) de la partie distale (2d) du corps (2).

11. Canule (1) selon l'une des revendications 1 à 10, dans laquelle le second conduit auxiliaire (7) comprend une section de forme oblongue au moins sur la partie distale (2d) du corps (2).

12. Canule (1) selon l'une des revendications 8 à 11, comprenant un conduit supplémentaire (10) formé dans la collerette (3) et s'étendant du premier conduit auxiliaire (6) jusqu'à deux orifices supplémentaires de sortie (11) réalisés dans une partie supérieure de la collerette (3), les deux orifices supplémentaires de sortie (11) étant disposés de manière à être en regard de chaque narine du patient lorsque la canule (1) est installée sur la patient.

13. Canule (1) selon l'une des revendications 1 à 12, dans laquelle au moins un des conduits auxiliaires (6, 7) comprend un orifice débouchant dans le conduit principal (5) possédant une forme tronconique, l'orifice ayant une section plus grande que la section moyenne du conduit auxiliaire (6, 7).

14. canule (1) selon l'une des revendications 4 à 13, dans laquelle chaque portion (63, 73) de conduit auxiliaire (6, 7) s'étendant radialement dans la collerette (3) forme un angle compris entre 0° et 20° et plus particulièrement un angle d'environ 10° avec le plan défini par la surface de la collerette (3) de manière à orienter les orifices d'entrée/sortie (64 et 74) vers le visage du patient.

## Patentansprüche

1. Oropharyngealkanüle (1), umfassend einen Körper (2), der aus einem gekrümmten distalen Teil (2d) zum Einführen in den Mund eines Patienten und zum Blockieren seiner Zunge und einem proximalen Teil (2p) gebildet ist, der einen Bund (3), der geeignet ist, auf den Lippen des Patienten zu liegen, eine Hauptleitung (5), die einen Fluiddurchgang zwischen dem proximalen Teil (2p) und dem distalen Teil (2d) des Körpers (2) bildet, und zwei Hilfsleitungen (6, 7) umfasst, die im Körper (2) gebildet sind und sich vom proximalen Teil (2p) bis in die Hauptleitung (5) erstrecken, **dadurch gekennzeichnet, dass** jede Hilfsleitung (6, 7) im Bund (3) in einer Eingangs-/Ausgangsöffnung (64, 74) mündet, die bezüglich der Achse (II - II') der Hauptleitung (5) in eine radiale Richtung ausgerichtet ist, so dass sie seitlich beiderseits des Mundes des Patienten ausgerichtet ist, wenn die Kanüle (1) im Patienten platziert ist.

2. Kanüle (1) nach Anspruch 1, wobei die Richtung zwischen jeder Eingangs-/Ausgangsöffnung (64, 74) und der Achse (II -II') der Hauptleitung (5) einen Winkel zwischen 0° und 30° und insbesondere zwischen 10° und 20° bildet, wobei eine Achse durch die Mundwinkel geht, wenn die Kanüle (1) im Patienten platziert ist.

3. Kanüle (1) nach einem der Ansprüche 1 oder 2, wobei jede Hilfsleitung (6, 7) eine an den Bund (3) gekoppelte Eingangs-/Ausgangsbiegung umfasst, wobei jede Hilfsleitung (6, 7) den Bund (3) bis in die Eingangs-/Ausgangsbiegung durchquert, die in der Eingangs-/Ausgangsöffnung (64, 74) mündet.

4. Kanüle (1) nach einem der Ansprüche 1 oder 2, wobei jede Hilfsleitung (6, 7) einen radial im Bund (3) gebildeten Abschnitt (63, 73) umfasst, wobei die Eingangs-/Ausgangsöffnung (64, 74) radial am Bund (3) angeordnet ist.

5. Kanüle (1) nach einem der Ansprüche 1 bis 4, umfassend zwei Injektions-/Extraktionsschläuche (8, 9), die geeignet sind, hinter den Ohren des Patienten vorbeigeführt zu werden, wenn die Kanüle (1) im Patienten platziert ist, wobei ein erstes Ende jedes Schlauchs mit einer Eingangs-/Ausgangsöffnung (64, 74) verbunden ist.

6. Kanüle (1) nach Anspruch 5, umfassend einen Klemmring, der geeignet ist, die beiden Schläuche (8, 9) zusammenzuhalten und an den beiden Schläuchen (8, 9) entlang zu gleiten, um die Kanüle (1) an Ort und Stelle zu halten, wenn sie im Patienten platziert und jeder Schlauch (8, 9) hinter einem Ohr vorbeigeführt ist.

7. Kanüle (1) nach einem der Ansprüche 5 oder 6, wobei ein zweites Ende der Injektions-/Extraktionsschläuche (8, 9) Mittel zum Anschluss an Injektions- oder Extraktionsapparate umfasst.

8. Kanüle (1) nach einem der Ansprüche 1 bis 7, wobei die erste Hilfsleitung (6) eine Eingangsöffnung (64) umfasst, die mit einem Schlauch (8) zur Injektion von Sauerstoff in die Atemwege des Patienten verbunden ist, und die zweite Hilfsleitung (7) eine Ausgangsöffnung (74) umfasst, die mit einem Schlauch (9) zur Extraktion von Gasen, die vom Patienten ausgeatmet worden sind, verbunden ist, um eine kapnometrische Messung durchzuführen.

9. Kanüle (1) nach Anspruch 8, wobei die zweite Hilfsleitung (7) im distalen Abschnitt (2d) mündet und die erste Hilfsleitung (6) im proximalen Abschnitt (2p) gegenüber dem Bund (3) mündet.

10. Kanüle (1) nach Anspruch 1 bis 8, wobei die erste Hilfsleitung (6) in einer oberen Wand (S) des distalen Teils (2d) des Körpers (2) gebildet ist und die zweite Hilfsleitung (7) in einer unteren Wand (I) des distalen Teils (2d) des Körpers (2) gebildet ist.

11. Kanüle (1) nach einem der Ansprüche 1 bis 10, wobei die zweite Hilfsleitung (7) mindestens am distalen Teil (2d) des Körpers (2) einen länglichen Querschnitt umfasst.

12. Kanüle (1) nach einem der Ansprüche 8 bis 11, umfassend eine im Bund (3) gebildete zusätzliche Leitung (10), die sich von der ersten Hilfsleitung (6) bis zu zwei zusätzlichen Ausgangsöffnungen (11) erstreckt, die in einem oberen Teil des Bunds (3) ausgeführt sind, wobei die beiden zusätzlichen Ausgangsöffnungen (11) so angeordnet sind, dass sie jedem Nasenloch des Patienten gegenüberliegen, wenn die Kanüle (1) im Patienten platziert ist.

13. Kanüle (1) nach einem der Ansprüche 1 bis 12, wobei mindestens eine der Hilfsleitungen (6, 7) eine Öffnung umfasst, die in der Hauptleitung (5) mündet und kegelstumpfförmig ist, wobei der Querschnitt der Öffnung größer als der mittlere Querschnitt der Hilfsleitung (6, 7) ist.

14. Kanüle (1) nach einem der Ansprüche 4 bis 13, wobei jeder sich radial im Bund (3) erstreckende Abschnitt (63, 73) der Hilfsleitung (6, 7) einen Winkel zwischen 0° und 20° und insbesondere einen Winkel von ungefähr 10° mit der durch die Oberfläche des Bunds (3) definierten Ebene bildet, so dass die Eingangs-/Ausgangsöffnungen (64 und 74) zum Gesicht des Patienten hin ausgerichtet sind.

## Claims

1. Oropharyngeal cannula (1) comprising a body (2) formed by a distal part (2d) which is curved in order to be inserted into a patient's mouth and to immobilize his tongue and by a proximal part (2p) comprising a collar (3) which can rest on the patient's lips, a main duct (5) forming a passage for fluid between the proximal part (2p) and the distal part (2d) of the body (2), and two auxiliary ducts (6, 7) which are formed in the body (2) and extend from the proximal part (2p) as far as into the main duct (5), **characterized in that** each auxiliary duct (6, 7) opens into the collar (3) over an input/output orifice (64, 74) which is oriented in a direction which is radial relative to the axis (II-II') of the main duct (5), such as to be oriented laterally on either side of the patient's mouth when the cannula (1) is installed on the patient.

2. Cannula (1) according to Claim 1, wherein the direction between each input/output orifice (64, 74) and the axis (II-II') of the main duct (5) forms an angle of between 0° and 30°, and more particularly between 10° and 20°, with an axis which passes via the corners of the lips when the cannula (1) is installed on the patient.

3. Cannula (1) according to either of Claims 1 and 2, wherein each auxiliary duct (6, 7) comprises an input/output bend which is coupled to the collar (3), each auxiliary duct (6, 7) passing through the collar (3) as far as into the input/output bend which opens over the input/output orifice (64, 74).

4. Cannula (1) according to either of Claims 1 and 2, wherein each auxiliary duct (6, 7) comprises a portion (63, 73) which is formed radially in the collar (3), the input/output orifice (64, 74) being disposed radially on the collar (3).

5. Cannula (1) according to one of Claims 1 to 4, comprising two flexible injection/extraction tubes (8, 9) which can be passed behind the patient's ears when the cannula (1) is installed on the patient, a first end of each tube being connected to an input/output orifice (64, 74).

6. Cannula (1) according to Claim 5, comprising a clamping ring which can hold the two tubes (8, 9) together and slide along the two tubes (8, 9) such as to hold the cannula (1) in place when it is installed on the patient and each tube (8, 9) passes behind an ear.

7. Cannula (1) according to either of Claims 5 and 6, wherein a second end of the injection/extraction tubes (8, 9) comprises means for connection to injection or extraction devices.

8. Cannula (1) according to one of Claims 1 to 7, wherein the first auxiliary duct (6) comprises an input orifice (64) which is connected to a tube (8) for injection of dioxygen into the patient's respiratory tracts, and the second auxiliary duct (7) comprises an output orifice (74) which is connected to a tube (9) for extraction of gases exhaled by the patient, in order to carry out a capnographic measurement.

9. Cannula (1) according to Claim 8, wherein the second auxiliary duct (7) opens into the distal portion (2d) and the first auxiliary duct (6) opens into the proximal portion (2p), opposite the collar (3).

10. Cannula (1) according to Claim 1 to 8, wherein the first auxiliary duct (6) is formed in an upper wall (S) of the distal part (2d) of the body (2) and the second auxiliary duct (7) is formed in a lower wall (I) of the distal part (2d) of the body (2).

11. Cannula (1) according to one of Claims 1 to 10, wherein the second auxiliary duct (7) comprises a cross section with an oblong form, at least over the distal part (2d) of the body (2).

12. Cannula (1) according to one of Claims 8 to 11, comprising an additional duct (10) formed in the collar (3) and extending from the first auxiliary duct (6) as far as two additional output orifices (11) provided in an upper part of the collar (3), the two additional output orifices (11) being disposed such as to be opposite each of the patient's nostrils when the cannula (1) is installed on the patient.

13. Cannula (1) according to one of Claims 1 to 12, wherein at least one of the auxiliary ducts (6, 7) comprises an orifice which opens into the main duct (5) and has a frusto-conical form, the orifice having a larger cross section than the mean cross section of the auxiliary duct (6, 7).

14. Cannula (1) according to one of Claims 4 to 13, wherein each portion (63, 73) of auxiliary duct (6, 7) which extends radially in the collar (3) forms an angle of between 0° and 20° and more particularly an angle of approximately 10° relative to the plane defined by the surface of the collar (3), such as to orient the input/output orifices (64 and 74) towards the patient's face.
